# EUROPEAN PATENT APPLICATION

(11) **EP 2 098 187 A1**
(43) Date of publication of application: **09.09.2009**
(21) Application number: 09250586.6
(22) Date of filing: 02.03.2009
(51) Int. Cl.: A61B 19/00

(54) **Intraluminal tissue markers**

(30) Priority: 03.03.2008 US 41374
(71) Applicant: Ethicon Endo-Surgery, Inc., Cincinnati, OH 45242 (US)
(72) Inventor: Voegele, James W., Cincinnati, Ohio 45245 (US)
(74) Representative: Tunstall, Christopher Stephen

(57) **Abstract**

Methods and devices are provided for marking tissue (20) to be subsequently located for removal from a body or for other examination. In general, a marker is provided that can be delivered to a target tissue (20). In one embodiment, the marker can include a solution (22) having a visual marking component and a palpably identifiable tactile marking component. The marker can remain in the body and be subsequently visually and/or palpably identified to locate the target tissue (20).

## Description

### FIELD OF THE INVENTION

The present invention relates to intraluminal tissue markers and methods for marking tissue intraluminally.

### BACKGROUND OF THE INVENTION

Colonoscopy is an outpatient procedure in which the rectum and the inside of the lower large intestine (colon) are examined. Colonoscopies are commonly used to evaluate bowel disorders, rectal bleeding or polyps (usually benign growths) found on contrast x-rays. Colonoscopies are also performed to screen people over age 50 for colon and rectal cancer. During a colonoscopy, a physician uses a colonoscope (a long, flexible instrument about 1/2 inch in diameter) to view the lining of the colon. The colonoscope is inserted through the rectum and advanced to the large intestine.

If necessary during a colonoscopy, small amounts of tissue can be removed for analysis (called a biopsy) and polyps can be identified and removed. In many cases, colonoscopy allows accurate diagnosis and treatment without the need for a major operation. However, in some cases the tissue cannot be removed during the colonoscopy, and thus must be removed in a subsequent surgical procedure. In these situations, india ink or blue dye is topically injected during the preoperative colonoscopy to mark the tumor site. However, such a procedure includes the intrinsic danger of possibly injecting dye into the peritoneal cavity. In addition, the injected marker may also spread so widely that the intended site may become obscured.

Accordingly, there remains a need for improved methods and devices for marking tissue, such as the bowel wall.

### SUMMARY OF THE INVENTION

The present invention generally provides methods and devices for marking tissue to be subsequently located for removal from a body or for other examination. In one aspect, a method for marking tissue is provided that includes delivering a marking solution to tissue. The marking solution can have a first component that forms a visible marking on a surface of the tissue and a second component that forms a palpably identifiable tactile marking on the tissue. The marking solution can have a variety of compositions. In some embodiments, at least one of the first and second components can include two chemicals that react to form a marking when the marking solution is delivered to the tissue. The marking solution can be delivered to tissue using a variety of devices, alone or in combination. The visible marking can be identified in a variety of ways, such as by being visible to the naked eye, with or without exposure to an excitation source. In some embodiments, the visible marking can have a wavelength in a non-visible range.

In another aspect, a method for marking tissue includes positioning a device containing a marking solution proximate to a target tissue in a patient's body and delivering the marking solution from the device to the target tissue to form a visual mark on the tissue with a first component of the marking solution and to form a palpably identifiable tactile marking on the target tissue with a second component of the marking solution. The marking solution can be delivered to the tissue in a variety of ways. For example, at least a portion of the marking solution can be delivered into a subdermal layer of tissue proximate to the target tissue. In some embodiments, delivering the marking solution from the device can cause the first and second components to mix together.

The method can also include removing the device from the patient's body and locating the target tissue by palpably identifying the tactile marking and/or by visually identifying the visual mark on the target tissue. Locating the target tissue can optionally include delivering energy to the target tissue to visually identify the marked tissue. In some embodiments, delivering energy to the target tissue can cause the visual mark to fluoresce.

In another aspect, a tissue marking system is provided. The system includes a marking solution containing a first component that can form a visible marking on a surface of tissue and a second component that can form a palpably identifiable tactile marking under the surface of the tissue. In some embodiments, the visible marking can have a wavelength in a non-visible range. The marking solution can have a variety of compositions. In one embodiment, at least one of the first and second components can include two chemical components that are mixed together to form a marking when the marking solution is injected into tissue. In an exemplary embodiment, the marking solution can include an active ester in N-methylpyrrolidone. In another embodiment, the first component can include an electrophile monomer in N-methylpyrrolidone and a dye, and the second component can include a nucleotide polymer in water.

In other aspects, the marking solution can be contained within a delivery device that can inject the marking solution into tissue. The delivery device can have a variety of configurations. For example, the delivery device can have a needle disposed at its distal end for injecting the marking solution into tissue. The delivery device can also have a first chamber containing the first component and a second chamber containing the second component.

### BRIEF DESCRIPTION OF THE DRAWINGS

The invention will be more fully understood from the following detailed description taken in conjunction with the accompanying drawings, in which:

FIG. 1 is a perspective view of one embodiment of an introducer device having a delivery device disposed therein that can deliver a marking solution to a tissue;

FIG. 2 is a perspective view of the delivery device of FIG. 1 delivering a marking solution to a tissue;

FIG. 3 is a perspective, partially cross-sectional view of one embodiment of a single-chamber syringe;

FIG. 4 is a perspective, partially cross-sectional view of one embodiment of a double-chamber syringe;

FIG. 5 is a perspective, partially cross-sectional view of one embodiment of a double-barrel syringe;

FIG. 6 is a perspective, partially cross-sectional view of one embodiment of a double-barrel syringe having a mixing chamber;

FIG. 7 is a cross-sectional schematic view of one embodiment of a needle disposed in a housing;

FIG. 8 is a cross-sectional schematic view of the needle of FIG. 7 extending beyond a distal end of the housing;

FIG. 9 is a schematic view of one embodiment of a needle tip;

FIG. 10 is a schematic view of another embodiment of a needle tip;

FIG. 11 is a perspective view of one embodiment of an introducer device having a delivery device disposed therein and delivering a marking solution to tissue;

FIG. 12 is a perspective view of the tissue of FIG. 2 with the marking solution delivered thereto;

FIG. 13 is a cross-sectional view of one embodiment of an introducer device disposed within a body lumen and a marker marking a tissue in the body lumen;

FIG. 14 is a cross-sectional view of the marker of FIG. 13 being palpably located in the body lumen; and

FIG. 15 is a diagram illustrating one embodiment of a laparoscopic system for viewing a fluorescent marker.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides methods and devices for marking a target tissue to be subsequently located for removal from a body, for diagnosis, for treatment, or for other purposes. While the methods and devices disclosed herein can be used in conventional, open surgical procedures and hand assisted laparoscopic surgery (HALS), they are particularly useful in minimally invasive surgical procedures, such as endoscopic and other laparoscopic procedures. The principles described herein can be applicable to the particular types of tools described herein and to a variety of other surgical tools having similar functions. In addition, the tools can be used alone in a surgical procedure, or they can be used in conjunction with other devices that facilitate the surgical procedure. A person skilled in the art will appreciate that the present invention has application in conventional open surgical instrumentation as well as application in robotic-assisted surgery.

In general, a marker is provided that can be delivered to a target tissue desirable for marking, e.g., for removal from a body or for other examination. The target tissue can include tissue to be treated, diagnosed, removed, geographically marked, or otherwise examined, as well as tissue proximate to tissue to be treated, diagnosed, removed, geographically marked, or otherwise examined. In an exemplary embodiment, the marker can include a solution that can form a visual marking and a palpably identifiable tactile marking on the target tissue. The marker can remain in the body and be subsequently visually and/or palpably identified to locate the target tissue. In this way, the marker can provide flexibility and ease in locating the target tissue because the marker can be identified using various techniques. Being able to identify the marker in more than one way can also provide identification confirmation and improve certainty in determining a marker's location. The visual and tactile markings can help improve chances of locating the target tissue because if one of the visual and tactile markings fades, reduces in area or volume, becomes bioabsorbed, becomes obscured or damaged by tissue or fluid in the body, "bleeds" into surrounding tissue, or changes in any other way to affect its ability to be located, then the other one of the markings can still be used to locate the target tissue. Furthermore, the marker can provide markings on both a surface of the target tissue and within tissue, e.g., in a subdermal layer, thereby improving the marker's chances of identification over time because the marker can be less likely to become obscured or damaged both on the tissue's surface and within the tissue. While the marker can be used to mark any tissue for any purpose, in an exemplary embodiment the marker is configured for delivery through the working channel of a delivery device and for use in marking tissue for removal from the body, e.g., a lesion, a polyp, or other tissue growth or unhealthy tissue identified during a colonoscopy and intended to be removed from the bowel wall during a subsequent surgical procedure. In another exemplary embodiment, the marker can be used to geographically mark tissue to indicate a surgical procedure site, e.g., a location of biopsied tissue. In yet another exemplary embodiment, two or more markers can be delivered to define a line or an area indicating the target tissue.

The marking solution can have a variety of compositions. In an exemplary embodiment the marking solution can include two components, one component to visually mark a tissue and another component to tactilely mark the tissue. The visually-marking and tactilely-marking components can each be formed from a variety of materials, preferably biocompatible materials safe for use in the body. The marking solution and its components are preferably fluids, although one or more of the marking solution and its components can include any combination of solids or partial solids (e.g., a gel), either before or after delivery to a target tissue.

The visually-marking component of the marking solution can include one or more materials configured to be applied to a tissue and be visually identifiable on, including through, the tissue's surface. The material used for the visually-marking component can be a one-part solution configured to provide a visual marking, or it can be a multiple-part solution including a dye added to another chemical. The dye can include any material configured to be visually identifiable on and/or through a tissue surface with or without application of energy, as discussed further below. Non-limiting examples of dye include D&C Violet No. 2, ink, or other visible color dye (e.g., a dye having a wavelength within the visible range, i.e., from about 400nm to 700nm), an infrared dye (e.g., a dye having a wavelength near or within the infrared range, i.e., from about 600nm to 1350nm), a fluorescent nanoparticle, and any other dye known in the art. Using a dye having a wavelength outside the visible range can help reduce chances of the dye obscuring the color or stasis of the target tissue. In an exemplary embodiment, the visually-marking component can include an electrophile monomer at 10% solids in N-methylpyrrolidone (NMP) and D&C Violet No. 2 incorporated into the electrophile monomer in NMP. Other non-limiting examples of the visually-marking component include D&C Violet No. 2 and a 10%, a 38%, or a 60% solution of an active ester compound in NMP, Cy 5.5 (fluorescing dye) manufactured by GE Healthcare, Chalfont St. Giles, United Kingdom, and Indocyanine Green (fluorescing dye) manufactured by Acros Organics N.V., Geel, Belgium.

In one exemplary embodiment, the visually-marking component can include a fluorescent nanoparticle, which may require light for visibility. A person skilled in the art will appreciate the fluorescent nanoparticles can be formed from a variety of materials using various methods. Exemplary fluorescent nanoparticles and methods for making the same are disclosed in detail in U.S. Application No. 11/771,490 of Voegele et al. filed June 28, 2007 and entitled "Nanoparticle Tissue Based Identification and Illumination," U.S. Publication No. 2004/0101822 of Wiesner et al. entitled "Fluorescent Silica-Based Nanoparticles," U.S. Publication No. 20046/0183246 of Wiesner et al. entitled "Fluorescent Silica-Based Nanoparticles," and U.S. Publication No. 2006/0245971 of Bums et al. entitled "Photoluminescent Silica-Based Sensors and Methods of Use," which are hereby incorporated by reference in their entireties. A person skilled in the art will also appreciate that fluorescent semiconductor nanocrystals, also referred to as quantum dots, can also be used with the various methods and devices disclosed herein.

The tactilely-marking component of the marking solution can also include one or more materials configured to be delivered to a tissue and be tactilely identifiable on the tissue's surface or through the tissue's surface. In an exemplary embodiment, the tactilely-marking component can include a nucleotide polymer in water, e.g., a nucleotide polymer at 10% solids in water. Other non-limiting examples of the tactilely-marking component include a 10% solution of amino dextran in water, a 15% solution of Di-lysine in water and triethylamine, and a 72.4% solution of Hexamethylene diamine in water.

By way of non-limiting example only, the marking solution, including both visually-marking and tactilely-marking components can include a first composition of a 10% solution of an active ester compound in NMP and D&C Violet No. 2, and a second composition of a 10% solution of amino dextran in water, with the first and second compositions mixed in a 1:1 volume or weight ratio. In another non-limiting example, the marking solution can include a first composition of a 38% solution of an active ester compound in NMP and D&C Violet No. 2, and a second composition of a 15% solution of Di-lysine in water and triethylamine. As still another non-limiting example, the marking solution can include a first composition including a 60% solution of an active ester compound in NMP and D&C Violet No. 2, and a second composition of a 72.4% solution of Hexamethylene diamine in water, with the first and second compositions mixed in a 7:2:1 ratio that can form a solid gel immediately upon mixing.

The visually-marking component and/or the tactilely-marking component can be adjusted or can include one or more additional components for achieving a desirable level of various properties, such as image enhancement, drug delivery, viscosity, drying time, reflectivity, fluorescence, contrast (transparency), bioabsorption, sterilization response, shelf life, storage conditions, biocompatibility, temperature response, sealant properties, bi-product properties, metering control, surface condition response, and/or adherence to, penetration of, or bonding to tissue.

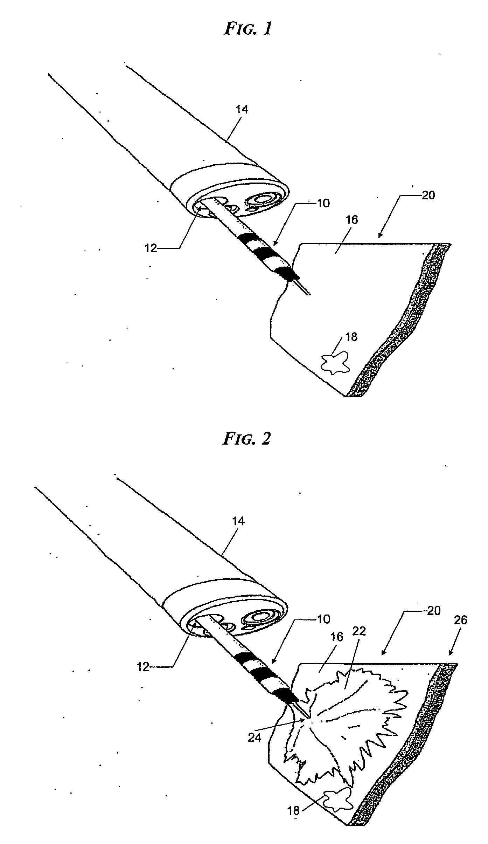

The marker can be delivered to a target tissue in a variety of ways. In an exemplary embodiment shown in FIG. 1, a delivery device 10 having a marking solution disposed therein can be advanced through a working channel 12 of an introducer device 14 and positioned proximate to a target tissue. While FIG. 1 illustrates the marker used to identify a tissue growth 18 on a surface 16 of a tissue 20, e.g., an organ, the marking solution can be used to mark any tissue anywhere within the body, e.g., within a tubular structure (such as the lower large intestine or any other body lumen), on and/or beneath a surface of an organ, etc. As shown in FIG. 2, a marking solution 22 can be delivered to the tissue 20 from a distal end 24 of the delivery device 10. The marking solution 22 can visually and tactilely mark the tissue 20 such that the marker can be identified in a plurality of ways. The marking solution 22 can be delivered to the tissue 20 in a variety of ways, such as by injection, application to a surface, and/or delivery in any other way appreciated by a person skilled in the art. The marking solution 22 can, but need not, penetrate the tissue's surface 16, e.g., into the tissue's subdermal layer 26. Furthermore, the marking solution 22 can be delivered to the tissue's surface 16 and at least partially naturally permeate into the tissue 20, e.g., into the subdermal layer 26 by absorption. In this way, the marking solution 22 can provide a surface marking on the tissue 20 and/or a marking under the surface 16 of the tissue 20, thereby allowing the tissue growth 18 to be located by one or both of the surface and sub-surface markings.

The marking solution 22 is illustrated as being delivered by the delivery device 10 advanced through the introducer device 14 and applied to the tissue's surface 16, but a variety of delivery devices and introducer devices can be used to deliver any amount of a marking solution to a tissue. In the event that the marker is application during a colonoscopy, the introducer can be any flexible, elongate colonoscope, endoscope, or other device that is capable of being inserted into the body, such as through a natural orifice, through a puncture hole formed in tissue, and in any other way appreciated by a person skilled in the art. The delivery device 10 can be any device that is effective to contain the marking solution and deliver it to the tissue 20. The delivery device 10 can also be configured to pass through the working channel 12 of the introducer device 14. However, in other embodiments, the delivery device 10 and/or introducer device 14 can be used alone to deliver the marking solution.

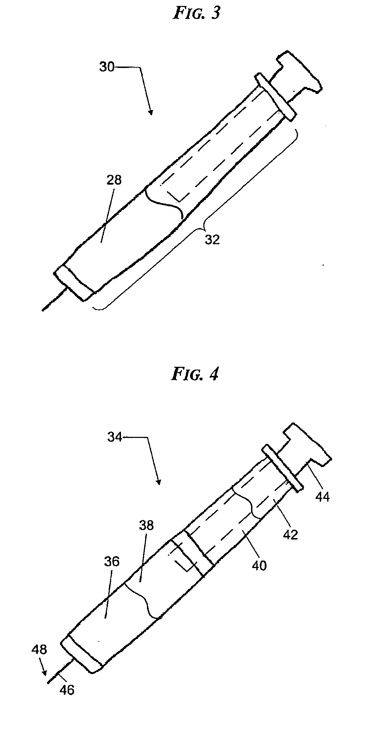

FIGS. 3-6 illustrate various exemplary delivery devices that can inject a marking solution onto or into tissue. FIG. 3 illustrates one embodiment of a single-chamber syringe 30 for delivering a marking solution 28 onto and/or into a tissue. Visually-marking and tactilely-marking components of the marking solution 28 can be delivered as a pre-mixed solution from the single-chamber syringe 30. The visually-marking and tactilely-marking components can be mixed together in any way outside the single-chamber syringe 30 and introduced into a barrel 32 of the single-chamber syringe 30, or the visually-marking and tactilely-marking components can be separately added to the single-chamber syringe 30 and mixed therein.

While not shown, the single-chamber syringe can include a static mixer, preferably substantially disposed within the syringe's barrel. The static mixer can have one or more static mixing components, e.g., a spiraled baffle or any other substantially non-moving parts appreciated by a person skilled in the art, that can mix one or more components of a marking solution disposed in the single-chamber syringe as the marking solution is distally pushed out of the syringe. The components of the marking solution can be fully or partially mixed by the static mixing components depending on one or more factors, such as size of the single-chamber syringe and composition of the marking solution.

FIG. 4 illustrates another embodiment of a delivery device for delivering a marking solution. In this embodiment the device is a double-chamber or dual-chamber syringe 34 having first and second chambers 38, 42. This allows two chemicals to be maintained in separate chambers and only mix when delivered, such by using a piston mechanism disposed between the chambers 38, 42 and actuated by movement of the syringe's plunger 44. Such a configuration is particularly desirable where the visually and tactilely marking components are configured to be disposed in separate chambers (or barrels, discussed below) and react with one another when mixed or where two chemicals are configured to react with one another to form a substantially solid mass that can be visually and tactilely located. The marking solution disposed in the double-chamber syringe 34 can include a first component 36 disposed in the first chamber 38 and a second component 40 disposed in the second chamber 42. One of the first and second components 36, 40 can include a tactilely-marking component while the other one of the first and second components 36, 40 can include a visually-marking component. When the double-chamber syringe's plunger 44 is distally advanced to eject the contents out of a needle 46 at the distal end 48 of the device, the first and second components 36, 40 can partially or fully mix together to be delivered from the needle 46 as at least a partially mixed solution.

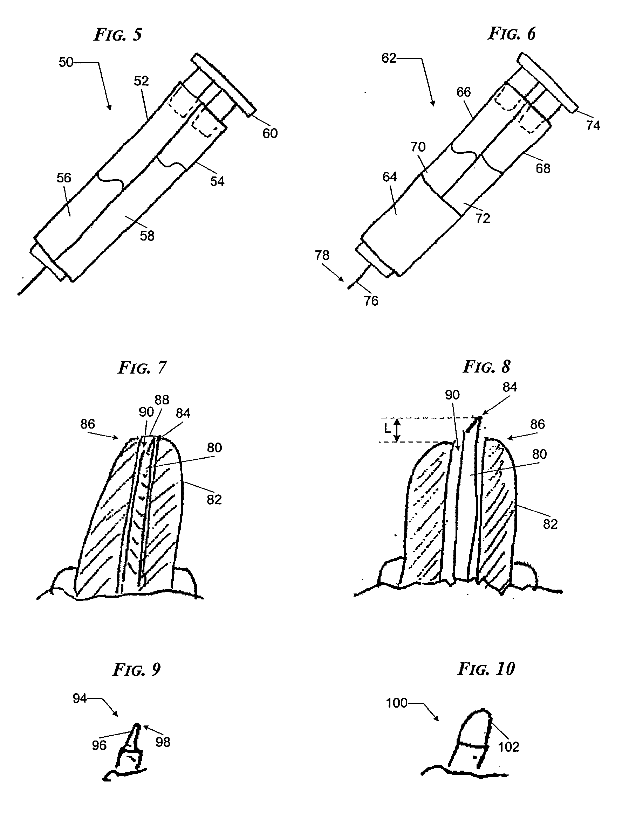

FIG. 5 illustrates another embodiment of a delivery device for delivering a marking solution to a tissue. In this embodiment, the device is a double-barrel syringe 50 having first and second barrels 52, 54 that re not in fluid communication with each other. The first and second barrels 52, 54 can include first and second solutions 56, 58 disposed respectively therein that can mix together when expunged from the double-barrel syringe 50, e.g., when the syringe's plunger 60 is distally advanced and the first and second solutions exit out of the syringe's needle. Although a single plunger 60 is illustrated, each of the barrels 52, 54 can include independent plungers and/or plungers configured to be coupled or de-coupled. One of the first and second solutions 56, 58 can include a tactilely-marking component while the other one of the first and second solutions 56, 58 can include a visually-marking component. The first and second barrels 52, 54 can have substantially the same or different volume, and substantially equal or different volumes of the first and second solutions 56, 58 (partially or fully filling their respective barrels 52, 54) can be disposed in the double-barrel syringe 50.

FIG. 6 illustrates still another embodiment of a delivery device in the form of a double-barrel syringe 62 having a mixing chamber 64, which can optionally include a static mixer. Similar to the double-barrel syringe 50 of FIG. 5, the double-barrel syringe 62 includes first and second barrels 66, 68 that can respectively have disposed therein first and second solutions 70, 72, one of which can include a tactilely-marking component while the other can include a visually-marking component. Alternatively, the barrels 66,68 can each include a chemical configured to react when mixed to form a visual and tactile marking. Distally advancing the syringe's plunger 74 can puncture or otherwise release a cap or a seal disposed between the barrels 66,68 and the mixing chamber 64 and push the first and second solutions 70, 72 into the mixing chamber 64 where the first and second solutions 70, 72 can at least partially mix before the first and second solutions 70, 72 are pushed out a needle 76 at the device's distal end 78. Although a single plunger 74 is illustrated, each of the barrels 66, 68 can include independent plungers and/or plungers configured to be coupled or de-coupled. The first and second barrels 66, 68 can have substantially the same or different volume, and substantially equal or different volumes of the first and second solutions 70, 72 (partially or fully filling their respective barrels 66, 68) can be disposed in the double-barrel syringe 62.

A person skilled in the art will appreciate that other delivery devices can be used to deliver the marking solution to tissue, and that the delivery device can vary in any number of ways from the delivery devices illustrated by way of non-limiting example in FIGS. 3-6. For example, a delivery device can include any number of chambers, include any number of barrels, have any number of plungers or other triggering mechanisms, etc. Furthermore, a delivery device can inject a marking solution onto and/or into tissue using a needle, through jet injection, or in any other way appreciated by a person skilled in the art.

The needle used to inject a marking solution can have any gauge and various configurations. For example, FIGS. 7 and 8 illustrate a retractable needle 80. The needle 80 is disposed within a housing 82 such that in a retracted position, shown in FIG. 7, a distal end 84 of the needle 80 is fully contained within the housing 82 and does not extend beyond a distal end 86 of the housing 82. The needle's housing 82 can be the shaft of the delivery device, or the housing 82 can be fixedly or removably coupled to a delivery device. The housing 82 can include a visually marking device similar to a tip of a marker or pen configured to apply a visually identifiable surface marking to a tissue when the needle's distal end 84 is fully contained within the housing 82. In an extended position, shown in FIG. 8, the needle's distal end 84 can extend a distance L beyond the housing's distal end 86. In other words, in the extended position, the needle 80 can penetrate into tissue to a depth equal to the distance L, thereby helping to ensure a desired marking depth and/or to ensure that the needle 80 does not puncture too far into or through a tissue, such as through a body lumen wall. The needle 80 can also be used to puncture through a tissue surface and/or to merely deliver a marking onto, rather than into, a tissue surface. The distance L vary, and it can be a controlled or variable value. For example, actuating a triggering mechanism of the delivery device can controllably advance the needle 80 a predetermined distance L beyond the housing's distal end 86, e.g., by pushing a button. Alternatively, actuating the triggering mechanism in varying degrees can advance the needle 80 any variable distance up, e.g., by depressing a plunger.

The needle 80 is preferably introduced into a body in the retracted position, thereby helping to prevent the needle 80 from puncturing, snagging, or otherwise contacting tissue or other foreign elements while being introduced into a body and placed in a desirable injection location. Furthermore, the distal end 86 of the housing 82 can be open or it can include a covering 88 disposed over the housing's distal end 90. The covering 88 can provide a barrier between the needle 80 and the surrounding environment, e.g., a body cavity, air outside a body, etc. The needle's distal end 84 can puncture, push through, or otherwise move the covering 88 and become exposed to the external environment when moved from the retracted position to the extended position.

The needle 80 can have an angled tip 92 as shown, however, the needle 80 (or any other delivery device needle) can have any shaped tip. For example, as shown in FIG. 9, a needle 94 can have a pointed tip 96. The pointed tip 96 can be substantially conical with a pointed or rounded distal end 98. In another embodiment, as shown in FIG. 10, a needle 100 can have a rounded tip 102.

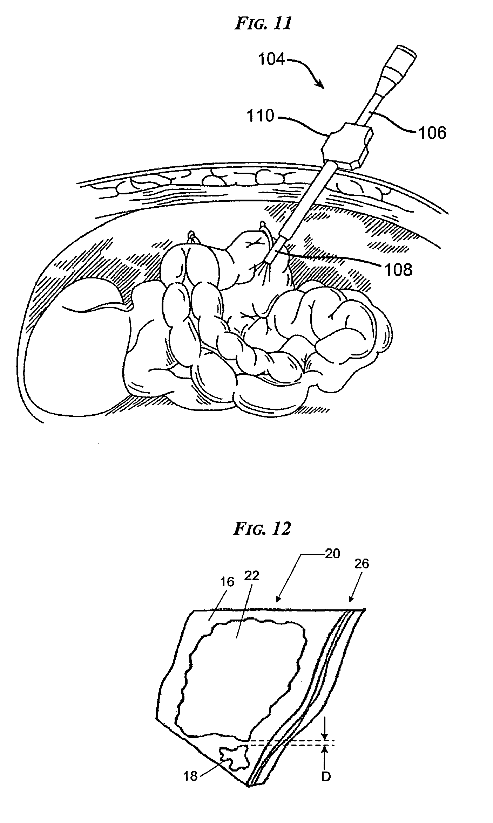

FIG. 11 illustrates another exemplary embodiment of a delivery device 104 that can apply a marking solution onto tissue. As shown, the delivery device 104 has an elongate shaft 106 with a distal tip 108. The elongate shaft 106 can have a variety of configurations, and the particular configuration can vary depending on the mode of insertion. In the illustrated embodiment, the elongate shaft 106 is disposed through an introducer, e.g., a cannula or a trocar 110, having a working channel that extends into a body cavity. The elongate shaft 106 can also include one or more lumens formed therein and extending between proximal and distal ends thereof. The lumen(s) can be used to deliver a marking solution to the distal tip 108. The distal tip 108 can also have a variety of configurations. In the illustrated embodiment, the distal tip 108 has a nozzle formed thereon for spraying the marking solution onto a tissue surface. In other embodiments, the distal tip 108 can include a brush for brushing the marking solution onto a tissue surface. Again, the particular configuration can vary depending on the intended use.

In use, the delivery device 104 can be inserted through the trocar 110, which is disposed through a tissue surface and into the abdominal cavity (or any other body cavity). As mentioned above, endoscopes or other introducer devices can also optionally be used, and/or the delivery device 104 can be an introducer device that is introduced directly through a natural orifice or through a man-made orifice. Once positioned adjacent to the target tissue, the delivery device 104 can be manipulated using, for example, controls to articulate the distal end of the delivery device 104 and/or controls to actuate the nozzle to deliver the marking solution to tissue.

A person skilled in the art will appreciate that a variety of other delivery devices known in the art can be used. By way of non-limiting example, U.S. Patent Appl. No. 11/533,506 of Gill et al., filed on September 20, 2006 and entitled "Dispensing Fingertip Surgical Instrument," which is incorporated herein by reference in its entirety, discloses one exemplary embodiment of a marking device.

A marking solution and/or a delivery device can be disposed within an introducer device at any point before or after the introducer device has been introduced into a body, including before or after the introducer device has been positioned at a desired position proximate to the target tissue. Preferably, the marking solution and/or delivery device is advanced through the introducer device's working channel after the tissue to be marked has been identified. Although, in some embodiments, the delivery device and/or the marking solution can be pre-loaded into the introducer device. Similarly, the marking solution can be disposed in the delivery device at any point before or after the delivery device has been advanced through the introducer device's working channel.

As illustrated in FIG. 12, once the marking solution 22 has been delivered to the tissue 20, the marker can remain on or in the tissue 20 proximate to the tissue growth 18 after any devices, such as the introducer device 14 and the delivery device 10, have been removed from the body. As mentioned above, the marker can be palpably and/or visually located on the tissue 20 to help locate the tissue growth 18. The marker can be configured to be palpably identified (e.g., located by touch) on the tissue 20, for example by touching the tissue surface 16 in which the marking solution has been injected, thus allowing the location of the tissue growth 18 to be determined. The marker can also be configured to be visually identified on the tissue 20. Visual observation of the marker can include observing the marker, observing one or more ridges along the tissue's surface 16, viewing still or moving images obtained by a scoping device, viewing an x-ray, viewing a barium image, viewing interaction with magnetic particles (if the marking solution 22 includes a magnetized component), tracing radiopharmaceuticals, etc. A person skilled in the art will appreciate that, as mentioned above, visual and/or palpable identification of the marker on the tissue 20 can include visual and/or palpable identification of the marker on or through the tissue's surface 16. A person skilled in the art will also appreciate that the marking solution 22 can be doped to be visible in multiple image modalities with a paramagnetic contrast agent such as ferric chloride, ferric ammonium citrate, and gadolinium-DTPA (with and without mannitol) for MRI applications, a short T1-relaxation agent such as mineral oil, oil emulsions, and sucrose polyester for MRI applications, a diamagnetic contrast agent for MRI applications, a superparamagnetic contrast agent such as magnetite albumin microspheres, oral magnetic particles, and superparamagnetic iron oxide (such as manufactured by AMAG Pharmaceuticals, Inc., Cambridge, Massachusetts) for MRI applications, a perfluorochemical for MRI applications, air aspiration to create bubbles for ultra-sound, and these or any other contrast agents alone or in combination for these or other applications, e.g., CT, PET, fluoroscopy, etc.

As previously indicated, the marking solution 22 can mark the tissue 20 proximate to the tissue growth 18 desired for marking, which includes a location where the marking solution 22 directly contacts the desired tissue 18 and/or a location where the marking solution 22 contacts the tissue 20 at a location adjacent to the tissue growth 18. As illustrated in FIG. 12, the marking solution 22 marks the tissue 20 at a shortest distance D from the tissue growth 18. The distance D can be zero or have any positive value, although the distance D is preferably of a value small enough such that any incision into or any examination of the tissue 20 at the location of the marker allows for relatively easy identification of the tissue growth 18. Once the marking solution 22 has been delivered proximate to the tissue growth 18, the distance D remains substantially unchanged until the marker begins bioabsorption or is bioabsorbed by the body, the marker is removed from the body, or the tissue growth 18 is removed from the body. In other words, the marker's position can be substantially static once the marker is delivered to the tissue 20. In this way, the marker can remain proximate to the tissue growth 18 and accurately mark the location of the tissue growth 18.

The marker can remain on the tissue 20 for any length of time, e.g., twenty-four hours, two days, one week, two weeks, one month, etc. Being safe for use in the body, the marker could remain on the tissue 20 indefinitely, but preferably, the marker is bioabsorbed after it has been used to locate the tissue growth 18. The length of time the marker remains on the tissue 20 can depend on any number of factors, such as the marker's material composition.

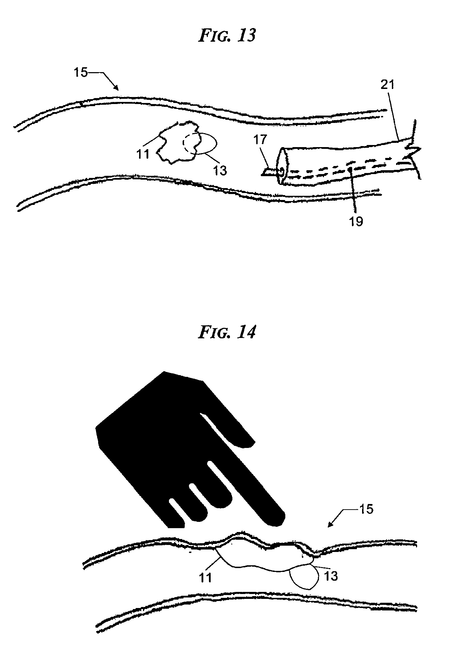

As shown in another embodiment of marker placement in FIG. 13, a distance between a marker 11 and a tissue growth 13 is zero with the marker 11 directly contacting the tissue growth 13. Here, the tissue growth 13 is formed within a body lumen 15. The marker 11 can also contact a n inner surface of the body lumen 15 proximate to the tissue growth 13. FIG. 13 also illustrates a delivery device 17 that is advanced through a working channel 19 of an introducer device 21 (e.g., a colonoscope) to deliver the marker 11 to the body lumen 15. The introducer device 21 and the delivery device 17 can be removed from the body lumen 15 (together or separately) after the marker is delivered, and the marker 11 can be palpably identified in the body lumen 15, as shown in FIG. 14, to help locate the desired tissue 13. The marker 11 can be palpably located directly, or the marker 11 can be palpably located through one or more layers of tissue adjacent to the body lumen 15, e.g., from outside a patient's body. As discussed above, the marker 11 can also or instead be visually located.

As mentioned above, when used in the body, light or other energy may need to be delivered to a tissue containing a marker to enable visual identification of the marker to locate the target tissue. The energy source can be external to the body for delivering energy internally, or an internal energy source can be used for internal application. Exemplary energy application methods and devices are described in U.S. Application No. 11/771,490 of Voegele et al. filed June 28, 2007 and entitled "Nanoparticle Tissue Based Identification and Illumination," mentioned above. In an exemplary embodiment, electromagnetic energy can be delivered to fluorescent nanoparticles disposed within a patient's body using a delivery apparatus, such as an endoscope or laparoscope. The delivery apparatus can be located externally, e.g., above a tissue surface, or internally. The excitation source can include any device that can produce electromagnetic energy at wavelengths that correspond to the absorption cross-section of the nanoparticles, including but not limited to, incandescent sources, light emitting diodes, lasers, arc lamps, plasma sources, etc. Various imaging technologies can also be used for detecting, recording, measuring or imaging fluorescent nanoparticles. In an exemplary embodiment, the imaging technology is adapted to reject excitation light, detect fluorescent light, form an image of the location of the nanoparticles, and transmit that image to either a storage or display medium. Exemplary devices include, for example, a flow cytometer, a laser scanning cytometer, a fluorescence micro-plate reader, a fluorescence microscope, a confocal microscope, a bright-field microscope, a high content scanning system, fiber optic cameras, digital cameras, scanned beam imagers, analog cameras, telescopes, microscopes and like devices.

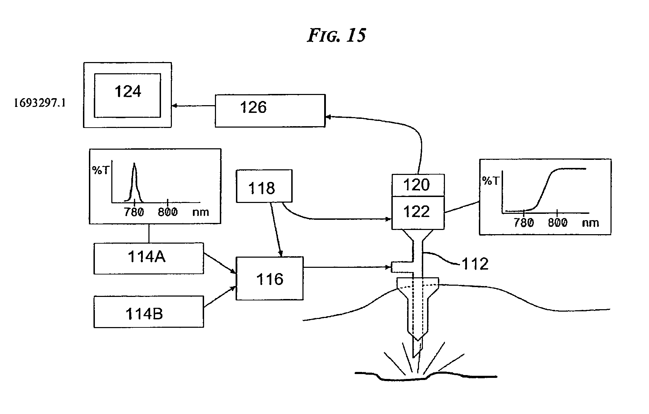

In an exemplary embodiment, the energy source is light, i.e., electromagnetic radiation, and the reading apparatus has an elongate shaft configured to be inserted into a body lumen and including a light emitting mechanism and an image receiving apparatus. Since fluorescent nanoparticles formed from a fluorophore core and a silica shell can absorb and emit energy in the visible, infrared, and near infrared frequencies, and they are illuminated at one wavelength and observed at a different shifted wavelength, it is desirable to provide an imaging apparatus that can enable visualization of such nanoparticles. FIG. 15 illustrates one exemplary embodiment of a laparoscope 112 that has two illumination or light emitting sources, generically illustrated as elements 114A, 114B. As shown, the laparoscope 112 utilizes an optical switch 116 to select the illumination source(s). One illumination source can be a standard white light source, such as a Xenon arc lamp used in standard endoscopic systems for illuminating and viewing in the visible spectrum. The second light source can be a narrow-band source associated with the absorbance cross-section of the nanoparticles, such as a laser, LED, mercury source, or filtered broadband source. One exemplary narrow-band source is a 780nm pigtailed laser diode. The optical switch 116 can connect the selected source 114A, 114B to an optical fiber bundle (not shown) that extends through the laparoscope 112 for transmitting the light through an eyepiece at the distal end of the laparoscope 112. When the light is transmitted, e.g., by depressing a switch, button, or foot pedal, generically illustrated as element 118, the fluorescent nanoparticles on the tissue will excite and fluoresce. The laparoscope 112 can also include an image receiving apparatus or camera 120 for collecting the reflected light from the fluorescent nanoparticles, and a filter switch 122 to place the appropriate optical filter between the eyepiece and the camera 120. The filter that is used for visualization of the nanoparticles, for example, must be highly efficient at rejecting the excitation wavelength in order to avoid saturation of the camera 120 while still being highly transparent at the wavelength of the emission of the nanoparticles. One exemplary filter is an interferometric long-pass filter with four orders of magnitude of rejection at the excitation wavelength and over 80% transmission at the peak of the fluorescent band.

As further shown in FIG. 15, the captured image can be transmitted to a monitor 124 coupled to the camera 120 by a camera control box 126. The monitor 124 can be an on-board monitor or an external monitor, as shown, or other reading devices can be used such as a readout display, an audible device, a spectrometer, etc. A person skilled in the art will appreciate that, while a laparoscope 112 is shown, various other elongate shafts, such as catheters and endoscopes, can be used to transmit and receive light for viewing fluorescent nanoparticles. The embodiment described illustrates real time viewing. A person skilled in the art will also appreciate that image(s) can be captured and stored for overlay transmission, such as showing a peristaltic pulse as a continuous path.

Additional utilization can also be achieved in the non-visible ranges, as previously indicated, by combining a visible light source with a non-visible light source enabling the ability to turn the non-visible image on or off. The images can be viewed either side by side or simultaneously by overlapping the images. The visible light source can vary and can be an ambient room source, an LED, a laser, a thermal source, an arc source, a fluorescent source, a gas discharge, etc., or various combinations thereof. The light source can also be integrated into the instrument or it can be an independent source that couples to the instrument.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam, and a liquid bath (e.g., cold soak).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

The following is a non-exhaustive list of embodiments of the invention that may be claimed in this application or in subsequently filed divisional applications:
1. A method for marking tissue, comprising:
   delivering a marking solution to tissue, the marking solution having a first component that forms a visible marking on a surface of the tissue and a second component that forms a palpably identifiable tactile marking on the tissue.
2. The method of embodiment 1, wherein the marking solution is delivered using a single delivery device.
3. The method of embodiment 1, wherein at least one of the first and second components includes two chemicals that react to form a marking when the marking solution is delivered to the tissue.
4. The method of embodiment 1, wherein the visible marking is visible with the naked eye.
5. The method of embodiment 1, wherein the visible marking is visible with the naked eye following exposure of the visible marking to an excitation source.
6. The method of embodiment 1, wherein the visible marking has a wavelength in a non visible range.
7. A method for marking tissue, comprising:
   positioning a device containing a marking solution proximate to a target tissue in a patient's body;
   delivering the marking solution from the device to the target tissue to form a visual mark on the tissue with a first component of the marking solution and to form a palpably identifiable tactile marking on the target tissue with a second component of the marking solution; and
   removing the device from the patient's body.
8. The method of embodiment 7, further comprising, after removing the device, locating the target tissue by visually identifying the visual mark on the target tissue.
9. The method of embodiment 8, wherein locating tissue includes delivering energy to the target tissue to visually identify the marked tissue.
10. The method of embodiment 9, wherein delivering energy to the target tissue causes the visual mark to fluoresce.
11. The method of embodiment 7, further comprising, after removing the device, locating the target tissue by palpably identifying the tactile marking.
12. The method of embodiment 7, wherein delivering the marking solution from the device causes the first and second components to mix together.
13. The method of embodiment 7, wherein at least a portion of the marking solution is delivered into a subdermal layer of tissue proximate to the target tissue.
14. A tissue marking system, comprising:
   a marking solution containing a first component configured to form a visible marking on a surface of tissue and containing a second component configured to form a palpably identifiable tactile marking under the surface of the tissue.
15. The system of embodiment 14, wherein the marking solution is contained within a delivery device configured to inject the marking solution into tissue.
16. The system of embodiment 15, wherein the delivery device has a needle disposed at its distal end for injecting the marking solution into tissue.
17. The system of embodiment 15, wherein the delivery device includes a first chamber containing the first component and a second chamber containing the second component.
18. The system of embodiment 14, wherein the visible marking has a wavelength in a non visible range.
19. The system of embodiment 14, wherein at least one of the first and second components includes two chemical components that are mixed together to form a marking when the marking solution is injected into tissue.
20. The system of embodiment 14, wherein the first component comprises an electrophile monomer in N methylpyrrolidone and a dye, and the second component comprises a nucleotide polymer in water.
21. The system of embodiment 14, wherein the marking solution includes an active ester in N- methylpyrrolidone.

## Claims

1. A tissue marking system, comprising:
a marking solution containing a first component configured to form a visible marking on a surface of tissue and containing a second component configured to form a palpably identifiable tactile marking under the surface of the tissue.

2. The system of claim 1, wherein the marking solution is contained within a delivery device configured to inject the marking solution into tissue.

3. The system of claim 2, wherein the delivery device has a needle disposed at its distal end for injecting the marking solution into tissue.

4. The system of claim 2, wherein the delivery device includes a first chamber containing the first component and a second chamber containing the second component.

5. The system of claim 1, wherein the visible marking has a wavelength in a non-visible range.

6. The system of claim 1, wherein at least one of the first and second components includes two chemical components that are mixed together to form a marking when the marking solution is injected into tissue.

7. The system of claim 1, wherein the first component comprises an electrophile monomer in N-methylpyrrolidone and a dye, and the second component comprises a nucleotide polymer in water.

8. The system of claim 1, wherein the marking solution includes an active ester in N-methylpyrrolidone.

9. A method for marking tissue, comprising:
delivering a marking solution to tissue, the marking solution having a first component that forms a visible marking on a surface of the tissue and a second component that forms a palpably identifiable tactile marking on the tissue.

10. A method for marking tissue, comprising:
positioning a device containing a marking solution proximate to a target tissue in a patient's body;
delivering the marking solution from the device to the target tissue to form a visual mark on the tissue with a first component of the marking solution and to form a palpably identifiable tactile marking on the target tissue with a second component of the marking solution; and
removing the device from the patient's body.
